Europäisches Patentamt

European Patent Office

Office européen des brevets

(19)

(11) Publication number: **0 347 256**
**A2**

# EUROPEAN PATENT APPLICATION

(21) Application number: **89306159.8**

(22) Date of filing: **16.06.89**

(51) Int. Cl.⁴: **G 01 N 33/58**
**G 01 N 33/537,**
**G 01 N 33/542, G 01 N 33/535**

(30) Priority: **17.06.88 JP 148017/88**
**09.11.88 JP 281426/88**

(43) Date of publication of application:
**20.12.89 Bulletin 89/51**

(84) Designated Contracting States: **BE DE FR GB SE**

(71) Applicant: **TOSOH CORPORATION**
**4560, Oaza-Tonda Shinnanyo-shi**
**Yamaguchi 746 (JP)**

(72) Inventor: **Daisuke, Miki**
**35-21, Sakuradai Midori-ku**
**Yokohama-shi Kanagawa (JP)**

**Mitoma, Yasutami**
**1-9-1, Minamidai**
**Sagamihara-shi Kanagawa (JP)**

**Kimio, Katsuura**
**1691, Nakanoshima Tama-ku**
**Kawasaki-shi Kanagawa (JP)**

**Inouye, Kuniyo**
**37-17, Sagamiono 7-chome**
**Sagamihara-shi Kanagawa (JP)**

(74) Representative: **Beresford, Keith Denis Lewis et al**
**BERESFORD & Co. 2-5 Warwick Court High Holborn**
**London WC1R 5DJ (GB)**

(54) Method for detecting a substance in a sample.

(57) A method for detecting a substance in a sample which comprises steps of:

(a) mixing a first enzyme complex comprising a first enzyme bound to a material having the affinity for the substance to be detected with the sample,

(b) separating the first enzyme complex bound to the substance to be detected (the first enzyme complex in bound form) from the first enzyme complex not bound to the substance to be detected (the first enzyme complex in free form),

(c) reacting the first enzyme complex in bound form with (i) a first substrate comprising a substrate for said first enzyme which is bound to a second enzyme; or (ii) a first substrate comprising a substrate to said first enzyme which is not bound to a second enzyme,

(d) removing the unreacted first substrate from the first product formed in step (i); or reacting the first product formed in step (ii) with a second enzyme complex comprising a second enzyme bound to a material having the affinity for said first product, and then removing the unreacted second enzyme complex,

(e) reacting the system with a second substrate to the second enzyme to form a second product, and

(f) detecting the second product produced in step (e).

Description

# A METHOD FOR DETECTING A SUBSTANCE IN A SAMPLE

## FIELD OF THE INVENTION

This invention relates to a method for detecting a substance in a sample by utilizing a two-stage enzyme reaction.

## BACKGROUND OF THE INVENTION

In the field of clinical diagnosis and immunodiagnosis, radioimmunoassay (RIA) using a radioisotope-labelled antibody and enzyme immunoassay (EIA) using an enzyme-labelled antibody are known.

RIA involves problems of storage and control of the radioisotopes used, the problem of detection accuracy arising from the stability of the radioisotopes, and the problem of the influence of radioisotopes on the human body.

EIA is free from the problems associated with RIA but has the limitation of a poor detection level. For example, when colorimetric analysis is used, the detection lower limit in the detection of peroxidase is 50 amol in a 10 minute assay or 5 amol in a 100 minute assay; that of $\beta$-D-galactosidase is 1,000 to 5,000 amol in a 10 minute assay or 100 to 500 amol in a 100 minute assay; and that of alkaline phosphatase is 2,000 to 10,000 amol in a 10 minute assay or 200 to 1,000 amol in a 100 minute assay, as reported by Eiji Ishikawa, Tadashi Kawai and Kiyoshi Miyai (ed.), Kosomeneki Sokuteiho (Enzyme Immunoassay in English), 3rd Ed., Igaku Shoin (1987). Such detection sensitivity is insufficient in the diagnosis of, for example, infectious diseases, and an assay system achieving higher sensitivity is needed.

## SUMMARY OF THE INVENTION

A primary object of this invention is to provide a detecting system for detecting a substance in a sample, which achieves higher detection sensitivity than the conventional EIA systems.

As a result of extensive investigations, inventors have established a detecting system utilizing a two-stage enzyme reaction.

The present invention provides a method for detecting a substance in a sample which comprises steps of:

(a) mixing a first enzyme complex comprising a first enzyme bound to a material having the affinity for the substance to be detected, with the sample,

(b) separating the first enzyme complex bound to the substance to be detected (the first enzyme complex in bound form) from the first enzyme complex not bound to the substance to be detected (the first enzyme complex in free form) to remove the first enzyme complex in free form,

(c) reacting the first enzyme complex in bound form with (i) a first substrate comprising a substrate for the first enzyme which is bound to a second enzyme; or (ii) a first substrate comprising a substrate for the first enzyme which is not bound to a second enzyme,

(d) removing the unreacted first substrate from the first product formed in step (c) - (i); or reacting the first product formed in step (c) - (ii) with a second enzyme complex comprising a second enzyme bound to a material having the affinity for the first product, and removing the unreacted second enzyme complex,

(e) reacting the resulting second enzyme containing complex with a second substrate for the second enzyme to form a second product, and

(f) detecting the second product produced in step (e).

In a first embodiment of the present invention, a method for detecting a substance in a sample comprises the steps of:

(a) mixing a first enzyme complex comprising a first enzyme bound to a material having the affinity for the substance to be detected, with the sample,

(b) separating the first enzyme complex bound to the substance to be detected (the first enzyme complex in bound form) from the first enzyme complex not bound to the substance to be detected (the first enzyme complex in free form) to remove the first enzyme complex in free form,

(c) reacting the first enzyme complex in bound form with a first substrate represented by the formula:

$X_1-Y_1-Z$

wherein $X_1$ is a second enzyme; $Y_1$ is a hapten or an antigen capable of undergoing an antigen-antibody reaction with an anti-(first product) antibody which is used in the subsequent step (d); Z is a modifying group inhibiting the antigen-antibody reaction between $Y_1$ and the anti-(first product) antibody; the $Y_1-Z$ bond is a bond cleavable by the first enzyme; and the $X_1-Y_1$ bond is a covalent bond,

(d) trapping (capturing) the first product formed in the step (c), which is represented by the formula:

$X_1-Y_1$

wherein $X_1$ and $Y_1$ are as defined above, with a immobilized anti-(first product) antibody,

(e) removing the unreacted first substrate,

(f) reacting the trapped first product with a second substrate to form a second product, and

(g) detecting the second product.

In the above first preferred embodiment, it is particularly preferred that the $Y_1-Z$ bond is an ester linkage, and $Y_1$ has a group capable of covalently bonding to $X_1$ at such a position which does not inhibit the antigen-antibody reaction with the anti-(first product) antibody; the $Y_1$-Z bond is a glycoside linkage, and $Y_1$ has a group capable of covalently bonding to $X_1$ at such a position which does not inhibit the antigen-antibody reaction with the anti-(first product) antibody; the $Y_1$-Z bond is an ether linkage, and $Y_1$ has a group capable of covalently bonding to $X_1$ at such a position which does not inhibit the antigen-antibody reaction with the anti-(first product) antibody; the $Y_1$-Z bond is an amide linkage, and $Y_1$ has a group capable of covalently bonding to $X_1$ at such a position which does not inhibit the antigen-antibody reaction with the anti-(first product) antibody; the first enzyme is carboxyl esterase, $Y_1$ is cortisol, and Z is a benzoyl group; the first enzyme is carboxyl esterase, $Y_1$ is cortisol and Z is phenylalanine; the first enzyme is phosphatase, $Y_1$ is cortisol and Z is a phosphoric acid radical; the first enzyme is carboxyl esterase, $Y_1$ is dopamine and Z is a benzoyl group; the first enzyme is carboxyl esterase, $Y_1$ is dopamine and Z is phenylalanine; the first enzyme is phosphatase, $Y_1$ is dopamine and Z is a phosphoric acid radical; the first enzyme is carboxyl esterase, $Y_1$ is testosterone and Z is a benzoyl group; the first enzyme is carboxyl esterase, $Y_1$ is testosterone and Z is phenylalanine; the first enzyme is phosphatase, $Y_1$ is testosterone and Z is a phosphoric acid radical; the first enzyme is β-D-galactosidase, $Y_1$ is cortisol and Z is a 1β-D-galactosyl group; the first enzyme is β-D-glucuronidase, $Y_1$ is cortisol and Z is a 1β-D-glucuronyl group; the first enzyme is β-D-glucosidase, $Y_1$ is cortisol and Z is a 1β-D-glycosyl group; the first enzyme is β-D-galactosidase, $Y_1$ is dopamine and Z is a 1β-D-galactosyl group; the first enzyme is β-D-glucuronidase, $Y_1$ is dopamine and Z is a 1β-D-glucuronyl group; the first enzyme is β-D-glycosidase, $Y_1$ is dopamine and Z is a 1β-D-glycosyl group; the first enzyme is β-D-galactosidase, $Y_1$ is testosterone and Z is a 1β-D-galactosyl group; the first enzyme is β-D-glucuronidase, $Y_1$ is testosterone and Z is a 1β-D-glucuronyl group; or the first enzyme is β-D-glycosidase, $Y_1$ is testosterone and Z is a 1β-D-glycosyl group. In a second embodiment of the present invention, the assay system for detecting a substance in a sample comprises steps of:

(a) mixing a first enzyme complex comprising a first enzyme bound to a material having the affinity for the substance to be detected, with the sample,

(b) separating the first enzyme complex bound to the substance to be detected (the first enzyme complex in bound form) from the first enzyme complex not bound to the substance to be detected (the first enzyme complex in free form) to remove the first enzyme complex in free form,

(c) reacting the first enzyme complex in bound form with a first substrate represented by the formula:

$Y_2 - Y_1 - Z$

wherein $Y_2$ is a hapten, an antigen or an antibody; $Y_1$ is a hapten or an antigen capable of undergoing an antigen-antibody reaction with an anti-(first product) antibody which is used in the subsequent step (d); Z is a modifying group inhibiting the antigen-antibody reaction between $Y_1$ and the anti-(first product) antibody; the $Y_1$-Z bond is a bond cleavable by the first enzyme; and the $Y_2$-$Y_1$ bond is a covalent bond,

(d) trapping (capturing) the first product obtained in step (c) which is represented by the formula:

$Y_2$-$Y_1$

wherein $Y_1$ and $Y_2$ are as defined above, with a immobilized anti-(first product) antibody,

(e) removing the unreacted first substrate,

(f) reacting the trapped first product with a compound represented by the formula:

$Y_3$-$X_2$

wherein $Y_3$ is a hapten, an antigen or an antibody capable of bonding to $Y_2$; $X_2$ is a second enzyme; and the $Y_3$-$X_2$ bond is a covalent bond, and removing the unreacted compound of the above formula,

(g) reacting the resulting second enzyme containing complex with a second substrate to the second enzyme, and

(h) detecting the second product produced in step (g).

In the above second preferred embodiment, it is particularly preferred that the $Y_1$-Z bond is an ester linkage, and $Y_1$ has a group capable of covalently bonding to $Y_2$ at such a position which does not inhibit the antigen-antibody reaction with the anti-(first product) antibody; the $Y_1$-Z bond is a glycoside linkage, and $Y_1$ has a group capable of covalently bonding to $Y_2$ at such a position which does not inhibit the antigen-antibody reaction with the anti-(first product) antibody; the $Y_1$-Z bond is an ether linkage, and $Y_1$ has a group capable of covalently bonding to $Y_2$ at such a position which does not inhibit the antigen-antibody reaction with the anti-(first product) antibody; the $Y_1$-Z bond is an amide linkage, and $Y_1$ has a group capable of covalently bonding to $Y_2$ at such a position which does not inhibit the antigen-antibody reaction with the anti-(first product) antibody; the first enzyme is carboxyl esterase, $Y_1$ is cortisol, and Z is a benzoyl group; the first enzyme is carboxyl esterase, $Y_1$ is cortisol and Z is phenylalanine; the first enzyme is phosphatase, $Y_1$ is cortisol and Z is a phosphoric acid radical; the first enzyme is carboxyl esterase, $Y_1$ is dopamine and Z is a benzyol group; the first enzyme is carboxyl esterase, $Y_1$ is dopamine and Z is phenylalanine; the first enzyme is phosphatase, $Y_1$ is dopamine and Z is a phosphoric acid radical; the first enzyme is carboxyl esterase, $Y_1$ is testosterone and Z is a benzoyl group; the first enzyme is carboxyl esterase, $Y_1$ is testosterone and Z is phenylalanine; the first enzyme is phosphatase, $Y_1$ is testosterone and Z is a phosphoric acid radical; the first enzyme is β-D-galactosidase, $Y_1$ is cortisol and Z is a 1β-D-galactosyl group; the first enzyme is β-D-glucuronidase, $Y_1$ is cortisol and Z is a 1β-D-glucuronyl group; the first enzyme is β-D-glucosidase, $Y_1$ is cortisol and Z is a 1β-D-glycosyl group; the first enzyme is β-D-galactosidase, $Y_1$ is dopamine and Z is a 1β-D-galactosyl group; the first enzyme is β-D-glucuronidase, $Y_1$ is dopamine and Z is a 1β-D-glucuronyl group; the first enzyme is β-D-glycosidase, Y₁ is dopamine and Z is a 1β-D-glycosyl group; the first enzyme is β-D-galactosidase, Y₁ is testosterone and Z is a 1β-D-galactosyl group; the first enzyme is β-D-glucuronidase, Y₁ is testosterone and Z is a 1β-D-glucuronyl group; or the first enzyme is β-D-glycosidase, Y₁ is testosterone and Z is a 1β-D-glycosyl group.

## DETAILED DESCRIPTION OF THE INVENTION

In carrying out the detection according to the present invention, a sample to be detected (e.g., a body fluid such as blood (e.g., blood serum or blood plasma), urine, etc.) is mixed with a first enzyme bound to a material (e.g., a nucleic acid, an antibody, an antigen, a ligand or an inhibitor) having the affinity for the substance (e.g., a nucleic acid, a protein, a peptide, a hormone or a drug) to be detected.

The typical examples of the substance to be detected include T3 (triiodothyronine), T4 (tyroxine), LH (lutenizing hormone), FSH (follicle stimulating hormone), TSH (thyroid stimulating hormone), TBG (thyroxine binding globulin), CT (calcitonin), PRL (prolactin), hCGA (human chorionic gonadotropin), Insulin, C-peptide, Cortisol, Aldosterono, ACTH (adrenocorticotropic hormone), Progesterone, CEA (carcinoembryonic antigen), CA19-9, AFP (α-fetoprotein), BFP (basic fetoprotein), Ferritin, β2-Microglobulin, TPA (tissue polypeptide antigen), γ-Seminoprotein, PAP (prostatic acid phosphatase), AIDS virus, CMV (cytomegalovirus), HAV (hepatitis A virus), HBV (hepatitis B virus), Non-A-Non-B virus, EVB (epstein barr virus), HSV (herpes simplex virus), Parvo virus, Staphyrococcus, Streptococcus, Mycobacterium, Neisseria gonorrhoeae, Salmonella, Pseudomonas, etc.

By the detection of the above-described substances, for example, the diagnoses of various infectious diseases or cancer become possible.

The material having the affinity for the substance to be detected may be selected depending on the properties of the substance, for example, when the substance to be detected is a hormone, a protein or a peptide as described above, an antibody obtained using these substances as antigen is used; when the substance to be detected is a microorganism (e.g., a specific DNA or RNA thereof) as described above or nucleic acid, the complementary nucleic acid is used; an inhibitor, e.g., a material of inhibiting an enzyme activity by bonding to a specific portion of enzymes such as L-isoleucine for L-threonine dehydrogenase, CANP (calcium-activated neutral protease), CANP inhibitor, etc., is used; and a ligand, e.g., a receptor for the bio-active substances (interleukin, interferon, etc. , is used.

Further, when the substance to be detected is an antibody for HTLV-III, the material having the affinity for the substance may be an HTLV-III antigen. Also, when the substance to be detected is an HBs antibody, an HBc antibody or an HBe antibody, the material having the affinity for the substance may be an HBs antigen, an HBc antigen or an HBe antigen.

It is preferred that the material having the affinity for the substance be an antibody.

The first enzyme used is an enzyme capable of converting the first substrate into a first product (e.g., the enzyme which can convert the first substrate which does not undergo an antigen-antibody reaction with a immobilized anti-(first product) antibody into a first product which undergoes an antigen-antibody reaction with the immobilized anti-(first product) antibody). Specific examples of such an enzyme include alkaline phosphatase, β-D-galactosidase, carboxyl esterase, and lipase.

The antibody specific to the substance to be detected in the sample can be prepared in a conventional manner as described, for example, in Köhler, Milstein, Nature, Vol. 256 (1975). It may be either polyclonal or monoclonal.

The material having the affinity for the substance and the first enzyme are conjugated each other to obtain the first enzyme complex. The first enzyme complex has a reactive activity with the substance to be detected and an activity of converting the first substrate into the first product. The binding of the first enzyme to the material having the affinity (e.g., antibody) can be effected in a conventional manner, for example, a maleimide method or a pyridyldisulfide method or a hinge method as described in Kato, K., Hamaguchi, Y., Fukui, H., Ishikawa, E., J. Biochem., Vol. 78, 235-237 and 423-425 (1975) and FEBS Lott., Vol. 56, 370-372 (1975), and Carlsson, J. et al., Biochem. J., Vol. 173, 723-737 (1978). When the substance to be detected is nucleic acid and the material having the affinity for the substance is the complementary nucleic acid, the first enzyme complex is mixed with a single-stranded nucleic acid and the mixture is stirred. In the case of a double-stranded nucleic acid, it is denatured to the single-stranded nucleic acid prior to mixing.

After the first enzyme complex (e.g., enzyme-labelled antibody) and the sample are mixed, the first enzyme complex in free form (not bound to the substance to be detected) can be removed, for example, by trapping the complex comprising the substance and the first enzyme complex with the material (having the affinity for the substance to be detected) immobilized on the inner wall of a reaction vessel or on the beads placed in a reaction vessel which is not inhibited by the reaction of the first enzyme complex to the substance, followed by B/F separation (bound-form/free-form separation) processing as described, for example, in U.S. Patent 4,816,408 which is employed in conventional enzyme immunoassay techniques. The labelled antibody in free form can be removed by trapping with an immobilized antibody which recognizes the substance to be detected at a site different from that reactive to the labelled antibody, followed by B/F separation (bound-form/free-form separation) employed in conventional enzyme immunoassay techniques.

The above trapper can be prepared by binding to a solid phase (e.g., the inner wall of reaction vessel or the beads placed in reaction vessel) in the chemical bonding method or physical adsorbing method, for example, as a method for honding an antibody to a solid phase, a chemical bonding method, a method of reacting a modifying group on the surface of the solid phase with a chemical group in the antigen as described in U.S. Patent Application No. 881, 692, and as a physical adsorbing method, a method of forming a solid phase using polystyrene having properties of adsorbing protein, and contacting the antibody thereto.

Removal of the first enzyme complex in free form (e.g. the labelled antibody in free form) antibody in free form can also be carried out by ge' filtration or centrifugation by using the difference of molecular weight between free form and bound form.

Then, a first substrate is reacted with the complex comprising the substance to be detected and the above first enzyme complex. The first substrate used in the present invention is present in two forms of (i) type comprising a substrate to the first enzyme which is bound to a second enzyme and (ii) type comprising a substrate to the first enzyme which is not bound to a second enzyme. The second enzyme used is an enzyme capable of converting a second substrate into a detectable second product. To assure high detection sensitivity, examples of preferred second enzymes are those having a great turnover (molecular active) number, such as alkaline phosphatase, $\beta$-D-galactosidase, glucose-6-phosphate dehydrogenase, glucose oxidase, and peroxidase. If the first enzyme and the second enzyme are the same, the first substrate undergoes the action of the second enzyme, i.e., the second enzyme in the first substrate converts the first substrate into the first product. Accordingly, the second enzyme should be different from the first enzyme.

The first product is produced by the reaction of the complex comprising the substance to be detected and the first enzyme complex, and the first substrate.

In the case that the first substrate of type (ii) described above is issued, the first product which is not composed of second enzyme is produced. In such a case, then a second enzyme complex comprising the second enzyme bound to a material having the affinity for the first product reacts with the first product.

After the preparation described above, the unreacted first substrate in the case of which the first substrate of type (i) described above is used or the unreacted second enzyme complex in the case of which the first substrate of type (ii) described above is used, is removed, for example, by trapping the first product or the complex comprising the first product and the second enzyme with the material immobilized (having the affinity for the first product) on the inner wall of a reaction vessel or on the beads placed in a reaction vessel which is not inhibited by the reaction of the first product and the material composed of the second enzyme complex in the case of the first substrate of type (ii) described above, followed by a B/F separation mentioned above. Finally, a second substrate to be converted into a second product by the second enzyme is reacted with the second enzyme included in the complex obtained through the above-mentioned preparation under reaction conditions as described, for example, in U.S. Patent Application Serial No. 898,946, filed on August 21, 1986 and European. Patent Publication No. 216,177, published on April 13, 1987, and then the resulting second product is measured.

The second substrate used in the present invention can be selected on the properties of the second enzyme to be used. The relationship between the second substrate and the second enzyme, and the typical examples of the second substrate and second enzyme are described, for example, in Robert H. Yolken, Review of Infectious Disease, Vol. 4, No. 1, January-February, 1982.

The detection of the second product can be carried out in accordance with the conventional techniques such as fluorometric analysis, colorimetric analysis, bioluminescent analysis or rate assay as described, for example, in U.S. Patent Application Serial No. 898,946, filed on August 21, 1986.

The first substrate used in the first preferred embodiment is a compound which can be converted by the action of the first enzyme into a first product capable of undergoing an antigen-antibody reaction with an anti-(first product) antibody. Such a substrate can be represented by the formula:

$X_1$-$Y_1$-$Z$

wherein $X_1$ is a second enzyme; $Y_1$ is a hapten (e.g., dopamine, cortisol, testosterone, etc.) or an antigen (e.g., insulin, angiotensine, glucagon, etc.) capable of undergoing an antigen-antibody reaction with an anti-first product) antibody used in step (d); $Z$ is a modifying group (e.g., a benzoyl group, a phenylalanine group, a phosphoric acid group, a 1$\beta$-D-galactosyl group, a 1$\beta$-D-glucuronyl group, a glucosyl group, etc.) inhibiting the antigen-antibody reaction between $Y_1$ and the anti-(first product) antibody; the $Y_1$-$Z$ bond is a bond cleavable by the first enzyme; and the $X_1$-$Y_1$ bond is a covalent bond.

Specific examples of the first substrate include a complex of the above formula wherein $X_1$ is alkaline phosphatase; $Y_1$ is dopamine; and $Z$ is a 1$\beta$-D-galactosyl group. When $Z$ is a benzoyl group, such includes a p-nitrobenzoyl group and a p-methoxybenzoyl group as well as an unsubstituted benzoyl group.

The first substrate used in the second preferred embodiment is a compound which can be converted by the action of the first enzyme into a first product capable of undergoing an antigen-antibody reaction with an anti-(first product) antibody. Such a substrate can be represented by the formula:

$Y_2$-$Y_1$-$Z$

wherein $Y_2$ is a hepten, an antigen, or an antibody; $Y_1$ is a hapten (e.g., dopamine, cortisol, testosterone, etc.) or an antigen (e.g., insulin, angiotensine, glucagon) capable of undergoing an antigen-antibody reaction with an anti-(first product) antibody used in step (d); and $Z$ is a modifying group (e.g., a benzoyl group, a phenylalanine group, a phosphoric acid group, a 1$\beta$-D-galactosyl group, a 1$\beta$-D-glucuronyl group, a glucosyl group, etc.) inhibiting the antigen-antibody reaction between $Y_1$ and the anti-(first product) antibody; the $Y_1$-$Z$ bond is a bond cleavable by the first enzyme; and the $Y_2$-$Y_1$ bond is a covalent bond.

Specific examples of the first substrate include a complex of the above formula wherein $Y_2$ is angiotensine II (3-8) (i.e., Val-Tyr-Ile-His-Pro-Phe); $Y_1$ is dopamine; and $Z$ is a 1$\beta$-D-galactosyl group.

When Z is a benzoyl group, the benzoyl group includes an unsubstituted benzoyl group, a p-nitrobenzoyl group, and a p-methoxybenzoyl group.

Synthesis of the first substrate in the first and second preferred embodiments will be described below using, for instance, 4-o-1β-galactosyl-dopamine-alkaline phosphatase complex wherein $X_1$ (second enzyme) is alkaline phosphatase; $Y_1$ is dopamine; and the $Y_1$-modifying group (Z) is a 1β-D-galactosyl group (used in the first embodiment) and 4-o-1β-galactosyl-dopamine-angiotensine II (3-8) complex in which $Y_1$ and Z are the same as above, and $Y_2$ is angiotensin II (3-8) (used in the second embodiment . These complexes are cleavable by β-D-galactosidase (first enzyme).

The phenolic hydroxyl group at the 4-position of N-trifluoroacetyldopamine, which can easily be synthesized by the N-trifluoroacetylation of dopamine, is galactosylated. This reaction can be carried out by using 1.0 to 1.5 equivalents of pentaacetylgalactose or tetraacetylgalactosyl-1-bromide in a dry organic solvent in the presence of a condensation catalyst. The condensation catalyst used includes mercury (II) salts, e.g., mercury (II) bromide and mercury (II) cyanide, trimethylsilyl trifluoromethanesulfonate, silver trifluoromethanesulfonate, and silver nitrate. The dry organic solvent used includes chloroform, dichloromethane, dioxane, nitromethane, acetonitrile, dimethylformamide, tetrahydrofuran, and diethyl ether. It is preferable, though not limitative, that the reaction be effected by using 1.5 equivalents of tetraacetylgalactosyl-1-bromide in dry acetonitrile in the presence of silver trifluoromethanesulfonate at a temperature of from -20°C to room temperature.

In order to remove the acetyl groups from the galactosyl moiety, the product is subjected to solvolysis by treatment with sodium methoxide in methanol to thereby yield 4-o-(1β-D-galactosyl)-N-trifluoroacetyldopamine. The resulting compound is purified by conventional silica gel column chromatography to separate by-products of the galactosylation, i.e., an isomeric compound having a galactosyl group at the 3-position and a 3,4-digalactosyl-substituted compound.

The compound is then subjected to a reaction to introduce a bond for connecting to alkaline phosphatase as a second enzyme ($X_1$) or angiotensin II (3-8) as $Y_2$ as follows. 4-o-(1β-D-galactosyl)-N-trifluoroacetyldopamine is subjected to de-trifluoroacetylation in an alkaline aqueous solution in an argon stream. The alkaline aqueous solution used includes an aqueous solution of sodium hydroxide, potassium hydroxide, sodium carbonate, potassium carbonate or ammonia. This reaction can be achieved, for example, in a 2 N sodium hydroxide aqueous solution at room temperature for a period of 24 hours. The reaction mixture is adjusted to a pH of 8.0 with dilute hydrochloric acid and then treated with a methanolic solution of N-succinimidyl 3-(2-pyridyldithio)propionate (hereinafter abbreviated as SPDP) to obtain 4-o-(1β-D-galactosyl)-N-[3-(2-pyridyldithio)propionyl]dopamine. Purification of the product by conventional silica gel column chromatography gives a colorless amorphous solid. With respect to the purity thereof, the content of 1α-galactosyl isomer in the product can be detected by proton NMR spectroscopic analysis, for example.

The compound can then be bound to alkaline phosphatase as a second enzyme ($X_1$) or angiotensine II (3-8) as $Y_2$ as follows. Alkaline phosphatase or angiotensine II (3-8) is treated with s-acetylmercaptosuccinic acid anhydride, and the resulting mercaptoalkaline phosphatase or mercapto-angiotensine (II) (3-8) is then treated with a methanolic solution of the above-prepared 4-o-(1β-D-galactosyl)-N-[3-(2-pyridyldithio)propionyl]dopamine in a phosphate buffer (pH = 7.0) in the presence of hydroxylamine at 4°C. There is thus obtained a 4-o-1β-galactosyldopamine-alkaline phosphatase complex or a 4-o-β-galactosyldopamine-angiotensine II (3-8) complex.

The thus prepared first substrate ($X_1$-$Y_1$-Z) or ($Y_2$-$Y_1$-Z) is reacted with a first enzyme, and, in step (d), the resulting first product ($X_1$-$Y_1$) or ($Y_2$-$Y_1$) is trapped by an immobilized anti-(first product) antibody. The anti-(first product) antibody to be used can be prepared according to known techniques as described, for example, in Köhler, Milstein, Vol. 156 (1975). The anti-(first product) antibody should not undergo an antigen-antibody reaction with a hapten or antigen with a modifying group being bonded thereto but should undergo an antigen-antibody reaction with a hapten or antigen from which the modifying group has been removed. So long as this requirement is met, any antibody, either monoclonal or polyclonal, may be employed. Fixing of the anti-(first product) antibody can be effected in a known manner, for example, by a physical adsorption method or a cross-linking method.

Removal of the unreacted first substrate can be carried out by a conventional B/F separation used in general EIA techniques.

The compound of the formula $Y_3$-$X_2$ which can be used in the second embodiment of the present invention includes a complex in which $Y_3$ is an anti-angiotensine II antibody and $X_2$ (second enzyme) is alkaline phosphatase in the case $Y_2$ is angiotensine II (3-8).

Removal of the unreacted compound ($Y_3$-$X_2$) from the reaction system can be effected by a conventional B/F separation used in general EIA techniques.

In the preparation of the compound $Y_3$-$X_2$, binding of the second enzyme ($X_2$) to the antibody ($Y_3$) can be carried out in a conventional manner. Binding of the second enzyme ($X_2$) to the hapten or antigen ($Y_3$) can be effected, for example, in accordance with the method employed for binding dopamine to angiotensine II (3-8) earlier given.

The second substrate which can be used in this invention is a compound capable of being converted by the action of the second enzyme into a second product which can be detected by fluorometric analysis, colorimetric analysis or bioluminescent analysis. For example, p-nitrophenyl phosphate or 4-methyl-umbelliferyl phosphate can be used in the case of using alkaline phosphatase as the second enzyme, 2-nitrophenyl-β-D-galactoside or 4-methyl-umbelliferyl-β-D-galactoside can be used in the case of using β-galactosidase as the second enzyme, and 1,2-phenylenediamine, 3,3',5,5',-tetramethylbenzidine, 4-hydroxyphenylacetate or 3-(4-hydroxyphenyl)propionic acid can be used in the case of using peroxidase as the second enzyme.

While the foregoing description has been directed to a two-stage enzyme reaction system for sensitivity amplification, it is possible to further amplify the detection sensitivity by a system involving more than two stages of enzyme reaction.

The. method of the present invention can be applied as a reagent kit for detecting a substance in a sample, comprising: a reaction vessel, a first enzyme complex comprising a first enzyme bound to a material having the affinity for the substance to be detected, another material having the affinity for the substance to be detected and not inhibiting the reaction between the first enzyme complex and the substance to be detected, which has been previously immobilized to a solid phase, and an anti-(first product) antibody having the affinity for the first product to be converted from the first substrate by the reaction with the first enzyme which has been previously immobilized to a solid phase.

Thus, in accordance with the present invention, a very low amount of virus particles or nucleic acid, which cannot be detected by a conventional enzyme immunoassay, can be detected with high sensitivity. Thus, the present invention is useful in the diagnosis of, for example, infectious diseases such as AIDS which can result even from even a slight amount of virus particles. As a result, early diagnosis and treatment, or a prevention of infection is made possible.

Further, since the two-stage enzyme immunoassay according to the present invention can provide higher detection sensitivity than conventional EIA systems, the amount of the sample to be detected, for example, the amount of blood to be collected from the subject, can be greatly reduced to 1/10 to 1/100 of the sampling amount (2 to 4 ml) in the usual enzyme immunoassay.

Further, since radioelements are not used, problems such as a storage stability and safety problems with such radioelements and loss of accuracy in detection due to the storage stability problem therewith can be avoided.

The present invention is now illustrated in greater detail by way of the following examples, but it should be understood that the present invention is not deemed to be limited thereto. In the examples, all percents are by weight unless otherwise indicated.

## EXAMPLE 1

### Detection of Insulin

Reagent:

An antibody (INSULIN MITSUI II, trade name, produced by Mitsui Seiyaku Kogyo K.K.) specific to insulin, to which β-D-galactosidase was bound as a first enzyme, was used to prepare an enzyme-labelled antibody (hereinafter referred to as enzyme-labelled antibody solution I).

For separation of the unreacted enzyme-labelled antibody, polystyrene balls to which an anti-insulin antibody, INSULIN MITSUI II, had been immobilized in a conventional manner were used (hereinafter referred to as antibody-immobilized ball I).

Preparation of First Substrate $X_1$-$Y_1$-Z, wherein $X_1$ is alkaline phosphatase; $Y_1$ is dopamine; and Z is a 1β-D-galactosyl group (hereinafter referred to as 1β-D-galactosyl-dopamine-alkaline phosphatase solution:

a) Synthesis of 4-o-(1β-D-galactosyl)-N-trifluoroacetyldopamine

In 15 ml of anhydrous acetonitrile were dissolved 518 mg (2.1 mmol) of N-trifluoroacetyldopamine and 1,564 mg (6.1 mmol) of silver trifluoromethanesulfonate, and 945 mg of powdered water-absorbing agent (4Å) (Molecular Sieves 4A 1/16, trade name, produced by Wako Pure Chemical Industries Limited) was added to the solution. To the resulting suspension was added (all at once) 1,352 mg (3.13 mmol) of tetraacetylgalactosyl bromide while stirring under ice cooling. The mixture was allowed to react under ice cooling for 10 minutes and then at room temperature for 22 hours while stirring. The reaction mixture was filtered, and the filtrate was concentrated. The residue was dissolved in dichloromethane, and the solution was washed successively with water, a 10% sodium bicarbonate aqueous solution, and water and dried over anhydrous magnesium sulfate. The solvent was removed by distillation, and the resulting oily substance was dissolved in 30 ml of absolute methanol. To the solution was added 30 ml of a 5 N methanolic solution of sodium methoxide, and the mixture was allowed to stand at room temperature in an argon stream for 18 hours. The reaction mixture was then neutralized with an ion exchange resin (IRC-50) to adjust it to a pH of 7.0, and concentrated aqueous ammonia was then added thereto to adjust the pH to 8.2, followed by filtration. The filtrate was concentrated, and the resulting oily substance was subjected to silica gel column chromatography using a mixed solvent of 15 v/v% methanol and chloroform (15 vol% methanol based on 100 vol% chloroform). There was thus obtained 287 mg of 4-o-(1β-D-galactosyl)-N-trifluoroacetyldopamine as a colorless oil.
IR Spectrum ν (KBr): 3352, 1710 cm$^{-1}$

b) Synthesis of 4-o-(1β-D-galactosyl)-N-[3-(2-pyridyldithio)propionyl]dopamine

In 2 ml of methanol was dissolved 30 mg (0.073 mmol) of 4-o-(1β-D-galactosyl)-N-trifluoroacetyldopamine, and 0.5 ml of a 2 N sodium hydroxide aqueous solution was added thereto, followed by allowing to stand at room temperature in an argon stream for 24 hours to thereby remove the trifluoroacetyl group. The reaction mixture was adjusted to a pH of 8.0 with 0.5 N hydrochloric acid, and a solution of 28'mg (0.090 mmol) of SPDP (N-succinimidyl-3-(2-pyridyldithio)propionate) in 1 ml of methanol was added thereto. The mixture was stirred at room temperature for 17 hours. The reaction mixture was diluted about 3 fold with a saturated sodium chloride aqueous solution, and extracted with a 1:1 (by volume) mixed solvent of 2-butanol and chloroform. The extract was dried over anhydrous magnesium sulfate, and the solvent was removed by distillation. The resulting colorless residue was purified by silica gel column chromatography using a mixed solvent of 15 v/v% methanol and chloroform (15 vol% methanol based on 100 vol% chloroform) to obtain 23 mg of 4-o-(1β-D-galactosyl)-N- [3-(2-pyridyldithio)propionyl]dopamine as a colorless solid.

IR Spectrum ν (KBr): 3380, 1635, 1419 cm⁻¹
Proton NMR Spectrum δ (d₄-MeOH):
2.56 (t, 2H, J = 7.3 Hz)
2.67 (t, 2H, J = 7.3 Hz)
3.01 (t, 2H, J = 6.8 Hz)
3.03-4.00 (m, 8H)
4.64 (d, 0.80H, J = 7.8 Hz, 1-H)
5.24 (d, 0.20H, J = 2.9 Hz, 1-H)
6.60-8.40 (m, 7H)

The proton NMR spectroscopic analysis revealed that the product was an isomeric mixture containing 20% of the 1α-galactosyl isomer.

c) Synthesis of
4-o-1β-D-galactosyl-dopamine-alkaline phosphatase

To a 0.15 mol/liter phosphate buffer (pH = 7.5) was added 5.5 ml of 5.45 mg/ml alkaline phosphatase. Separately, 174.2 mg of s-acetylmercaptosuccinic acid anhydride was dissolved in 1 ml of dioxane, and a 300 μℓ portion of the solution was added to the above prepared alkaline phosphatase solution, followed by incubation at 30°C for 1 hour. The solution was subjected to dialysis against 5 liters of a 0.1 mol/liter phosphate buffer (pH = 7.0) for one night. To the resulting solution were added a solution of 17.1 mg of 4-o-(1β-D-galactosyl)-N-[3-(2-pyridyldithio)propionyl]dopamine in 0.5 ml of methanol and 180 μℓ of a solution prepared by dissolving 14 mg of hydroxylamine in 200 μℓ of a 0.1 mol/liter phosphate buffer (pH = 7.0), followed by allowing to stand at 4°C overnight. The reaction mixture was concentrated to 2 ml by the use of an ultrafiltration membrane ("PM10" produced by Amicon Co.), and to the concentrate was added 10 ml of a 0.1 mol/liter phosphate buffer (pH = 7.0) containing 0.8% sodium chloride, 0.02% potassium chloride, 0.5% bovine serum albumin, and 0.1% sodium azide.

Preparation of Solutions:

The following solutions were prepared:

a) A 90 mg/ml sodium chloride aqueous solution (hereinafter referred to as cleaning solution I).

b) A 0.1 mol/liter phosphate buffer solution (pH = 7.0) containing 0.8% sodium chloride, 0.02% potassium chloride, and 0.05% Tween 20 (hereinafter referred to as cleaning solution II).

c) A solution consisting of 9.3 mg of p-nitrophenyl phosphate as a second substrate and 5 ml of a 0.5 mol/liter 2-amino-2-methyl-1-propanol acetate buffer (pH = 10.0) (hereinafter referred to as substrate solution).

d) A 0.14 mol/liter phosphate buffer (pH = 9.1) containing 0.1 mol/liter EDTA, and 0.1% sodium azide (hereinafter referred to as a reaction terminating solution).

Preparation of Anti-Dopamine Antibody-Immobilized Microtiter Plate:

An anti-dopamine antibody was immobilized to the inner wall of a microtiter plate through adsorption as follows. In each of 96 wells of a microtiter plate (product of Nunk Co.) there was placed 100 μℓ of 4.5 μg/ml anti-rabbit immunoglobulin G and the plate was allowed to stand at 4°C overnight. The well was washed three times with 300 μℓ portions of a solution comprising a 0.1 mol/liter phosphate buffer (pH = 7.0), 0.8% sodium chloride, and 0.02% potassium chloride. An anti-dopamine antibody POLYCLONAL ANTISERA Anti-dopamine for 0.05 ml use (produced by Immunotec Co.) in a vial was thoroughly shaken together with 10 ml of ion exchanged water, and 100 μℓ of the resulting solution was added to each well, followed by allowing to stand at 4°C overnight. Then, 250 μℓ of a 0.1 mol/liter phosphate buffer (pH = 7.0) containing 0.8% sodium chloride, 0.02% potassium chloride, 0.5% bovine serum albumin, and 0.1% sodium azide was added to each well, followed by allowing to stand at 4°C overnight. The thus prepared plate was designated as an antibody-immobilized plate.

Detection if Insulin:

Insulin in sample solutions was detected by using the above-described reagents, solutions, and the microtiter plate as follows.

a) A standard insulin solution of INSULIN MITSUI II was diluted with ion exchanged water to prepare an insulin solution at a concentration of 0, 10⁻¹, 10⁻², 10⁻³ or 10⁻⁴ μU/ml. In a test tube there was put 100 μℓ each of the insulin solutions, and 280 μℓ of enzyme-labelled antibody solution I was added to each of the test tubes at regular intervals between addition to one test tube and next addition to another test tube, and mixed. One of the antibody-immobilized balls I was added to each of the test tubes in the same order and at the same intervals as in the addition of enzyme-labelled antibody solution I. After confirming that the antibody-immobilized ball was completely soaked in the solution, the test tube was gently shaken and incubated at 37°C for 2 hours.

b) The whole amount of the reaction mixture in each test tube was removed by suction with an aspirator in the same order and at the same intervals as in a) above, and 2 ml of cleaning solution I was put in each test tube while washing the inner wall, followed by gentle shaking, and the cleaning solution was removed by suction. This cleaning procedure was repeated twice.

c) To each of the test tubes was added 100 μℓ of a 2.5 mg/ml solution of 1β-D-galactosyl-dopamine-alkaline phosphatase, followed by incubation at 45°C for 1 hour.

d) The reaction solution was put in the antibody-immobilized plate in an amount of 100 $\mu\ell$ per well using a microsyringe, followed by allowing the same to stand at 4°C overnight.

e) The well was washed five times with 300 $\mu\ell$ portions of cleaning solution II.

f) Into each of the wells was added 100 $\mu\ell$ of the substrate solution, followed by incubation at 37°C for 30 minutes. The reaction was stopped by the addition of 100 $\mu\ell$ of the reaction terminating solution.

g) The absorbance at 405 nm of the reaction system was then detected.

The results of the above assay are shown in Table 1.

TABLE 1

| Insulin Concentration ($\mu$U/ml) | Absorbance (405 nm) | S/N Ratio |
|---|---|---|
| $10^{-2}$ | 2.24 | 1.38 |
| $10^{-3}$ | 2.27 | 1.40 |
| $10^{-4}$ | 1.87 | 1.15 |
| 0 (ion exchanged water) | 1.63 | 1.00 |

It was established by the results of Table 1 that the assay system according to the present invention enables one to detect insulin at a level of $10^{-4}$ $\mu$U/ml ($7.2 \times 10^{-20}$ mol).

COMPARATIVE EXAMPLE

Insulin was detected with a commercially available insulin determination kit (INSULIN MITSUI II) based on the sandwich method.

1) An insulin solution was diluted with ion exchanged water to prepare an insulin solution having a concentration of 0, $10^{-1}$, $10^{-2}$, $10^{-3}$ or $10^{-4}$ $\mu$U/ml. In a test tube was put 100 $\mu\ell$ of each of the insulin solutions, and 280 $\mu\ell$ of enzyme-labelled antibody solution I was added to each of the test tubes at regular intervals between addition to one test tube and the next addition to another test tube and mixed. One of the antibody-immobilized balls I was placed in each test tube in the same order and at the same intervals as in the addition of enzyme-labelled antibody solution I. After confirming that the antibody-immobilized ball I was completely soaked in the solution, the solution was gently shaken and incubated at 37°C for 2 hours.

2) The whole amount of the reaction solution in each test tube was removed by suction using an aspirator in the same order and at the same intervals as in 1) above. In each test tube there was put 2 ml of cleaning solution I while washing the inner wall, followed by gentle shaking. The cleaning solution was removed by suction. This cleaning procedure was repeated twice.

3) In each test tube there was placed 0.5 ml of the substrate solution in the same order and at the same intervals as in 1) above. After gentle shaking, the system was incubated at 37°C for 1 hour. The reaction was stopped by the addition of 2.0 ml of the reaction terminating solution in the same order and at the same intervals as in above, and the absorbance at 420 nm was then detected.

The results of the detection are shown in Table 2.

TABLE 2

| Insulin Concentration ($\mu$U/ml) | Absorbance (405 nm) | S/N Ratio |
|---|---|---|
| 10 | 0.0596 | 2.18 |
| 1 | 0.0315 | 1.15 |
| $10^{-1}$ | 0.0285 | 1.04 |
| $10^{-2}$ | 0.0293 | 1.07 |
| $10^{-3}$ | 0.0272 | 1.00 |
| 0 (ion exchanged water) | 0.0273 | 1.00 |

As is shown in Table 2, insulin at a level of $10^0$ $\mu$U/ml ($7.2 \times 10^{-6}$ mol) could be detected by the conventional enzyme immunoassay technique.

EXAMPLE 2

Detection of Ferritin

Reagent:

An antibody (IR-1400 FERRITIN MITSUI, trade name, produced by Mitsui Seiyaku Kogyo K.K.) specific to ferritin, to which $\beta$-D-galactosidase was bound as a first enzyme, was used to prepare an enzyme-labelled antibody (hereinafter referred to as enzyme-labelled antibody solution II).

Polystyrene balls to which an anti-ferritin antibody, IR-1400 FERRITIN MITSUI, had been immobilized in a conventional manner were used for separation of the unreacted enzyme-labelled antibody (hereinafter referred to as antibody-immobilized ball II.

Preparation of First Substrate $Y_2$-$Y_1$-Z, wherein $Y_2$ is angiotensine II (3-8); $Y_1$ is dopamine; and Z is a 1$\beta$-D-galactosyl group (hereinafter referred to as 1$\beta$-D-galactosyl-dopamine-angiotensine II (3-8) solution:

a) 4-o-(1$\beta$-D-galactosyl)-N-trifluoroacetyldopamine was synthesized in the same manner as described in Example 1.

b) 4-o-(1$\beta$-D-galactosyl)-N-[3-(2-pyridyldithio)propionyl]dopamine was synthesized in the same manner as in Example 1.

c) Synthesis of 4-o-1β-D-galactosyl-dopamineangiotensine II (3-8):

In 1 ml of ethanol was dissolved 0.5 mg of angiotensine II (3-8), and 20 µℓ of a solution prepared by dissolving 174.2 mg of s-acetylmercaptosuccinic acid anhydride in 1 ml of dioxane was added to the angiotensine II solution. After incubation at room temperature for 1 hour, the solution was collected using DEAE-2SW (trade name, produced by TOSHO Corp.).

To 2 ml of the resulting solution were added 50 µℓ of a solution prepared by dissolving 27.0 mg of 4-o-(1β-D-galactosyl)-N-[3-(2-pyridyldithio)propionyl]dopamine in 1 ml of methanol and 40 µℓ of a solution prepared by dissolving 14 mg of hydroxylamine in 200 µℓ of a 0.1 mol/liter phosphate buffer (pH = 7.0). After being allowed to stand at room temperature overnight, the solution was collected using DEAE-2SW.

Preparation of Solutions:

Cleaning solutions I and II, substrate solution, and reaction terminating solutions were prepared in the same manner as in Example 1.

A solution of anti-angiotensine II-alkaline phosphatase at a concentration of 32.5 µg/ml was prepared in a conventional manner.

Preparation of Anti-Dopamine-Immobilized Microtiter Plate:

An anti-dopamine-immobilized microtiter plate was prepared in the same manner as in Example 1.

Detection of Ferritin:

a) A standard solution of ferritin ("IR-1400 FERRITIN MITSUI", produced by Mitsui Seiyaku Kogyo K.K.) was diluted with ion exchanged water to prepare a ferritin solution having a concentration of 0, $10^{-14}$, $10^{-15}$, $10^{-16}$, $10^{-17}$, $10^{-18}$, or $10^{-19}$ mol/50 µℓ. Into a test tube there was put 300 µℓ of enzyme-labelled antibody solution II, and the antibody-immobilized ball II was put therein one by one at regular intervals. Then, 50 µℓ each of the above prepared ferritin solutions was added to each test tube in the same order and at the same intervals as for the addition of the antibody-immobilized ball II. After confirming that the antibody immobilized ball was completely soaked in the solution, the system was gently shaken and incubated at 37°C for 2 hours.

b) The whole amount of reaction mixture in each test tube was removed by suction using an aspirator in the same order and at the same intervals as in a) above. To each test tube there was added 2 ml of cleaning solution I while washing the inner wall, followed by gentle shaking, and the cleaning solution was removed by suction. This cleaning procedure was repeated twice.

c) In each well of the antibody-immobilized plate there was placed 100 µℓ of the 1β-D-galactosyl-dopamine-angiotensine II solution, and each of the antibody-immobilized balls II having passed through the above steps a) and b) was put in the wells, followed by standing at room temperature overnight.

d) The well was washed twice with 300 µℓ portions of cleaning solution II.

e) In each of the wells there was placed 100 µℓ of the 32.5 µg/ml anti-angiotensine II-alkaline phosphatase solution, followed by standing at room temperature for one night.

f) The antibody-immobilized plate was washed twice with 300 µℓ portions of cleaning solution II.

g) The substrate solution (100 µℓ) was then added to each well and incubated at 37°C for 30 minutes. The reaction was stopped by the addition of 100 µℓ of the reaction terminating solution.

h) The absorbance at 405 nm was then measured.

The results obtained are shown in Table 3. It can be seen that the assay of this example enables one to detect $10^{-18}$ mol of ferritin.

TABLE 3

| Ferritin Concentration (mol) | Absorbance (405 nm) | S/N Ratio |
|---|---|---|
| $10^{-14}$ | 1.371 | 15.8 |
| $10^{-15}$ | 0.405 | 4.66 |
| $10^{-16}$ | 0.124 | 1.43 |
| $10^{-17}$ | 0.120 | 1.38 |
| $10^{-18}$ | 0.111 | 1.28 |
| 0 (ion exchanged water) | 0.087 | 1.00 |

EXAMPLE 3

Detection of β2-Microglobulin

Reagent:

β-D-Galactosidase-bound antibody specific to β2-microglobulin was prepared using IR-1500 β2-MG MITSUI (produced by Mitsui Seiyaku Kogyo K.K.). This reagent was designated enzyme-labelled antibody solution III.

For separation of the unreacted enzyme-labelled antibody, polystyrene balls to which an anti-β2-microglobulin antibody, IR-1500 β2-MG MITSUI, had been immobilized were used (hereinafter designated antibody-immobilized balls III).

1β-D-galactose-dopamine-angiotensine II (3-8), an anti-dopamine antibody, a second substrate, an anti-angiotensine II-alkaline phosphatase solution, cleaning solutions I and II, and an antibody-immobilized plate were prepared in the same manner as in Example 2.

$\beta_2$-Microglobulin was assayed in the same manner as in Example 2, and the results obtained are shown in Table 4.

It can be seen that the assay system of this example enables one to detect $10^{-18}$ mol of $\beta_2$-microglobulin.

TABLE 4

| $\beta_2$-MG Concentration (mol) | Absorbance (405 nm) | S/N Ratio |
|---|---|---|
| $10^{-15}$ | 0.492 | 2.08 |
| $10^{-16}$ | 0.309 | 1.31 |
| $10^{-17}$ | 0.270 | 1.14 |
| $10^{-18}$ | 0.243 | 1.03 |
| 0 (ion exchanged water) | 0.236 | 1.00 |

While the invention has been described in detail and with reference to specific embodiments thereof, it will be apparent to one skilled in the art that various changes and modifications can be made therein without departing from the spirit and scope thereof.

**Claims**

1. A method for detecting a substance in a sample which comprises steps of:

(a) mixing a first enzyme complex comprising a first enzyme bound to a material having the affinity for the substance to be detected with the sample,

(b) separating the first enzyme complex bound to the substance to be detected (the first enzyme complex in bound form) from the frist enzyme complex not bound to the substance to be detected (the first enzyme complex in free form),

(c) reacting the frist enzyme complex in bound form with (i) a first substrate comprising a substrate for said first enzyme which is bound to a second enzyme; or (ii) a first substrate comprising a substrate for said first enzyme which is not bound to a second enzyme,

(d) removing the unreacted first substrate from the first product formed in step (c)-(i); or

reacting the first product formed in step (c)-(ii) with a second enzyme complex comprising a second enzyme bound to a material having the affinity for said first product, and removing the unreacted second enzyme complex,

(e) reacting the system with a second substrate to the second enzyme to form a second product, and

(f) detecting the second product produced in step (e).

2. A method as claimed in claim 1, wherein said sample is a body fluid, a nucleic acid, a protein, a peptide, a hormone or a drug.

3. A method as claimed in claim 2, wherein said body fluid is blood, urine, blood serum, blood plasma, a nucleic acid, an antibody, an antigen, a ligand or an inhibitor.

4. A method as claimed in any preceding claim, wherein the separation and removal procedures in steps (b) and (d) are carried out by utilizing the difference of molecular weight between the reaction product and the unreacted substance to be removed.

5. A method as claimed in claim 4, wherein said procedures are carried out in accordance with a chromatrographic method.

6. A method as claimed in any preceding claim, wherein said separation procedure in step (b) is carried out in the presence of another material having the affinity for the substance to be detected and not inhibiting the reaction between the first enzyme complex and the substance to be detected, which has been previously immobilized to a solid phase, and/ or said removal procedure in step (d) is carried out in the presence of another material having the affinity for the first product but not having the affinity for the first substrate, which has been previously immobilized to a solid phase.

7. A method as claimed in claim 6, wherein said material having the affinity is an antibody.

8. A method as claimed in any preceding claim, wherein said detection of the second product is detected by a fluorometric, luminescent or colorimetric analysis.

9. A method as claimed in any preceding claim, wherein said first substance is a substrate represented by the formula:

$X_1 - Y_1 - Z$

wherein $X_1$ is a second enzyme; $Y_1$ is a hapten or an antigen capable of undergoing an antigen-antibody reaction with an anti-(first product) antibody which is used in a subsequent trapping step; Z is a modifying group inhibiting the antigen-antibody reaction between $Y_1$ and the anti-(first product) antibody; the $Y_1$- Z bond is a bond cleavable by the first enzyme; and the $X_1 - Y_1$ bond is a covalent bond; and

said unreacted first substrate is removed after trapping the first product formed in step (i), which is represented by the formula:

$X_1 - Y_1$

wherein X1 and Y1 are as defined above, with an anti-(first product) antibody previously immobilized to a solid phase with is specific to $Y_1$.

10. A method as claimed in any of claims 1 to 8, wherein said first substrate is a substrate represented by the forumla:

$Y_2 - Y_1 - Z$

wherein $Y_2$ is a hapten, an antigen or an antibody; $Y_1$ is a hapten or an antigen capable of undergoing an antigen-antibody reaction with an anti-(first product) antibody which is used in a subsequent trapping step; Z is a modifying group inhibiting the antigen-antibody reaction between $Y_1$ and the anti-(first product) antibody; the $Y_1$-Z bond is a bond cleavable by the first enzyme; and the $Y_2$-$Y_1$ bond is a covalent bond;

said second enzyme complex is a compound represented by the formula:

$Y_3$-$X_2$

wherein $Y_3$ is a hapten, an antigen or an antibody capable of bonding to $Y_2$; $X_2$ is a second enzyme; and the $Y_3$-$X_2$ bond is a covalent bond; and

the unreacted first substrate is removed after trapping the first product obtained in step (ii) which is represented by the formula:

$Y_2$-$Y_1$

wherein $Y_1$ and $Y_2$ are as defined above, with an immobilized anti-(first product) antibody previously immobilized to a solid phase which is specific to $Y_1$.

11. A method as claimed in claim 9, wherein: the $Y_1$ - $Z$ bond is an ester, glycoside, ether or amide linkage; and $Y_1$ has a group capable of covalently bondling to $X_1$ at such a position which does not inhibit the antigen-antibody reaction with the anti-first product antibody.

12. A method as claimed in claim 9, wherein:

(a) the first enzyme is carboxyl esterase, $Y_1$ is cortisol, and $Z$ is a benzoyl group;

(b) the first enzyme is carboxyl esterase, $Y_1$ is cortisol and $Z$ is phenylalanin;

(c) the first enzyme is phosphatase, $Y_1$ is cortisol and $Z$ is a phosphoric acid radical;

(d) the first enzyme is carboxyl esterase, $Y_1$ is dopamine and $Z$ is a benzoyl group;

(e) the first enzyme is carboxyl esterase, $Y_1$ is dopamine and $Z$ is phenylalanine;

(f) the first enzyme is phosphatase, $Y_1$ is dopamine and $Z$ is a phosphoric acid radical;

(g) the first enzyme is carboxyl esterase, $Y_1$ is testosterone and $Z$ is a benzoyl group;

(h) the first enzyme is carboxyl esterase, $Y_1$ is terstosterone and $Z$ is phenylalanin;

(i) the first enzyme is phosphatase, $Y_1$ is testosterone and $Z$ is a phosphorc acid radical;

(j) the first enzyme is β-D-galactosidase, $Y_1$ is corisol and $Z$ is a 1β-D-galactosyl group;

(k) the first enzyume is β-D-glucuronidase, $Y_1$ is cortisol and $Z$ is a 1β-D-glucuronyl group;

(l) the first enzyme is β-D-glycosidase, $Y_1$ is cortisol and $Z$ is a 1β-D-glycosyl group;

(m) the first enzyme is β-D-galactosidase, $Y_1$ is dopamine and $Z$ is a 1β-D-galactosyl group;

(n) the first enzyme is β-D-glucuronidase, $Y_1$ is dopamine and $Z$ is a 1β-D-glucuronyl group;

(o) the first enzyme is β-D-glycosidase, $Y_1$ is dopamine and $Z$ is a 1β-D-glycosyl group;

(p) the first enzyme is β-D-galactosi-

dase, $Y_1$ is testosterone and $Z$ is a 1β-D-galactosyl group;

(q) the first enzyme is β-D-glucuronidase, $Y_1$ is testosterone and $Z$ is a 1β-D-glucuronyl group; or

(r) the first enzyme is β-D-glycosidase, $Y_1$ is testosterone and $Z$ is a 1β-D-glycosyl group.

13. A method as claimed in claim 10, wherien the $Y_1$ -$Z$ bond is an ester, glycoside, ether, amide linkage, and $Y_1$ has a group capable of covalently bonding to $Y_2$ at such a position which does not inhibit the antigen-antibody reaction with the anti-(first product) antibody.

14. A method as claimed in claim 10, wherein:

(a) the first enzyme is carboxyl esterase, $Y_1$ is cortisol, and $Z$ is a benzoyl group;

(b) the first enzyme is carboxyl esterase, $Y_1$ is cortisol and $Z$ is phenylalanine;

(c) the first enzyme is phosphatase, $Y_1$ is cortisol and $Z$ is a phosphoric acid radical;

(d) the first enzyme is carboxyl esterase, $Y_1$ is dopamine and $Z$ is a benzoyl group;

(e) the first enzyme is carboxyul esterase, $Y_1$ is dopamine and $Z$ is phenylalanine;

(f) the first enzyme is phosphatase, $Y_1$ is dopamine and $Z$ is a phosphoric acid radical;

(g) the first enzyme is carboxyl esterase, $Y_1$ is testosterone and $Z$ is a benzoyl group;

(h) the first enzyme is carboxyl esterase, $Y_1$ is testosterone and $Z$ is phenylalanine;

(i) the first enzyme is phosphatase, $Y_1$ is testosterone and $Z$ is a phophoric acid radical; or

(j) the first enzyme is β-D-galactosidase, $Y_1$ is cortisol and $Z$ is a 1β-D-galactosyl group;

(k) the first enzyme is β-D-glucuronidase, $Y_1$ is cortisol and $Z$ is a 1β-D-glucuronyl group;

(l) the first enzyme is β-D-glucosidase, $Y_1$ is cortisol and $Z$ is a 1β-D-glycosyl group;

(m) the first enzyme is β-D-glactosidase, $Y_1$ is dopamine and $Z$ is a 1β-D-galactosyl group;

(n) the first enzyme is β-D-glucuronidase, $Y_1$ is dopamine and $Z$ is a 1β-D-glucuronyl group;

(o) the first enzyme is β-D-glycosidase, $Y_1$ is dopamine and $Z$ is a 1β-D-glycosyl group;

(p) the first enzyme is β-D-glactosidase, $Y_1$ is testosterone and $Z$ is a 1β-D-galactosyl group;

(q) the first enzyme is β-D-glucuronidase, $Y_1$ is testosterone and $Z$ is a 1β-D-glucuronyl group.

(r) the first enzyme is β-D-glycosidase, $Y_1$ is testosterone and $Z$ is a 1β-D-glycosyl group.

15. A reagent kit for detecting a substance in a sample, comprising:

(1) a reaction vessel,

(2) a first enzyme complex comprising a first enzyme bound to a material having the affinity for the substance to be detected,

(3) another material having the affinity for the substance to be detected and not inhibiting the reaction between the first enzyme complex and the substance to be detected, which has been previously immobilized to a solid phase, and

(4) an anti-(first product) antibody having the affinity for the first product to be converted from the first substrate by the reaction with the first enzyme which has been previously immobilized to a solid phase.

16. A reagent kit as claimed in claim 15, wherein said material having the affinity for the substance to be detected or said another immobilized material having the affinity for the substance to be detected is a nucleic acid, an antibody or an antigen.

17. A reagent kit as claimed in claim 15 or 16, wherein said solid phase is present in the form of beads.

18. A reagent kit as claimed in claim 15 or 16, wherein said solid phase is present on the inner wall of the reaction vessel.

19. A method of enzyme immounassay which comprises forming a first enzyme complex with a test substance and forming a second enzyme complex from the first complex, the second enzyme complex having a capacity for sensitivity amplification.